# FASCICULE DE BREVET EUROPEEN

(11) **EP 4 185 256 B1**
(45) Date de publication et mention de la délivrance du brevet: **10.07.2024**
(21) Numéro de dépôt: 21749671.0
(22) Date de dépôt: 12.07.2021
(51) Int. Cl.: A61F 9/009, A61B 90/25

(54) **DISPOSITIF D'APLANATION DESTINÉ À ÊTRE COUPLÉ À UN SYSTÈME DE CHIRURGIE OPHTALMOLOGIQUE LASER**
APPLANATIONSVORRICHTUNG ZUR KOPPLUNG AN EIN OPHTHALMOLOGISCHES LASERCHIRURGIESYSTEM
APPLANATION DEVICE INTENDED TO BE COUPLED TO AN OPHTHALMOLOGICAL LASER SURGERY SYSTEM

(30) Priorité: 22.07.2020 FR 2007710
(43) Date de publication de la demande: 31.05.2023
(73) Titulaire: UNIVERSITE DE BORDEAUX, 33000 Bordeaux (FR); CENTRE NATIONAL DE LA RECHERCHE SCIENTIFIQUE (CNRS), 75016 Paris (FR); Centre Hospitalier Universitaire de Bordeaux, 33400 Talence (FR)
(72) Inventeur: LOPEZ, John, 33170 GRADIGNAN (FR); IRIBARREN, Donetsi, 33270 FLOIRAC (FR); TOUBOUL, David, 33000 BORDEAUX (FR)
(74) Mandataire: Plasseraud IP
(86) Numéro de dépôt international: PCT/FR2021/051288
(87) Numéro de publication internationale: WO 2022/018349

(56) Documents cités:
- US-A1- 2009 069 794
- US-A1- 2013 165 911

## Description

### Domaine technique

L'invention relève du domaine des appareils de chirurgie oculaire. Plus précisément, l'invention concerne un dispositif d'aplanation destiné à aplanir l'œil du patient et à le maintenir en position pendant une opération de chirurgie ophtalmologique laser.

L'invention concerne également un appareil de chirurgie ophtalmologique destiné par exemple à la réalisation des greffes de cornées et la chirurgie réfractive oculaire équipé d'un dispositif d'aplanation associé.

### Technique antérieure

La cornée fait partie de la tunique périphérique de l'œil. C'est un milieu diélectrique transparent comme le verre, taillé en coupole à faces presque parallèles formant un dioptre asphérique biconvexe. Son diamètre moyen est d'environ 12 mm et son épaisseur moyenne centrale est d'environ 540 microns. Elle présente un rayon de courbure moyen de l'ordre 7,8 mm. Elle permet de faire converger les rayons lumineux incidents vers la rétine pour y former une image.

Sous l'effet d'un accident de la vie ou d'une pathologie spécifique, la cornée peut s'opacifier partiellement ou totalement, dégradant ainsi la vision. La greffe de cornée constitue alors le seul traitement efficace pour restituer la fonction de la cornée malade. Elle permet en outre de rétablir une bonne acuité visuelle et de supprimer les douleurs provoquées par les lésions cornéennes. La kératoplastie, ou greffe de cornée consiste généralement à extraire une partie ou la totalité d'une cornée pathologique, et à la remplacer par une cornée saine, provenant d'un donneur.

Il est également possible de faire appel à la chirurgie réfractive pour compenser les défauts de la vision, dits amétropies, traduisant d'une défaillance de focalisation des rayons lumineux sur le plan de la rétine à travers la rétine. Les amétropies opérables sont la myopie, l'hypermétropie, l'astigmatisme et la presbytie. Les lasers sont actuellement utilisés pour assister le chirurgien à venir traiter la cornée saine pour corriger les défauts de visions.

On peut citer ici l'exemple de la technique du LASIK qui consiste à découper superficiellement un volet de cornée d'une épaisseur de 90 à 120 microns à l'aide d'un microkératome mécanique (lame) ou d'un laser femtoseconde pour ensuite soulever ce volet le temps de remodeler la cornée par dessous. Le laser Excimer type ArF avec un rayonnement ultraviolet à 193nm et une durée d'impulsion de 10 à 25ns permet un remodelage très précis (environ 0,25 microns de photoablation par impulsion). Cette procédure est la plus pratiquée actuellement pour traiter la myopie.

Grâce aux développements importants dans la technologie de laser, la chirurgie oculaire laser est devenue une technique de choix dans les applications chirurgicales ophtalmologiques.

Lors de l'opération, afin d'assurer un couplage précis entre le faisceau laser et une région à traiter par le faisceau laser de l'œil, il est nécessaire de maintenir l'œil du patient en position fixe par rapport au faisceau laser de manière à éviter tout mouvement de l'œil par rapport au point focal du faisceau laser qui pourrait induire des résultats non optimaux ou des dommages irréversibles de l'œil.

Il est connu d'utiliser un dispositif d'aplanation qui est conçu pour venir au contact de la cornée et permettre d'aplanir la surface de celle-ci pendant l'opération pour permettre la focalisation du faisceau laser à une profondeur choisie. Il comprend généralement un cône d'aplanation et un anneau de succion. Le cône d'aplanation comprend une extrémité couplée à une extrémité terminale du système laser et une autre extrémité munie d'un verre d'aplanation. Le verre d'aplanation est maintenu en position, par exemple par collage sur un pourtour de l'extrémité du cône d'aplanation. Pendant l'opération, l'anneau de succion est engagé et centré par le chirurgien au niveau du limbe cornéen. Un système d'aspiration relié à l'anneau de succion permet d'assurer une immobilisation de l'oeil. Le cône d'aplanation est déplacé via la terminale du système laser pour venir s'engager dans l'anneau de succion de sorte que le verre d'aplanation soit en contact avec l'œil et assujettisse l'œil à la courbure de la lentille d'aplanation pendant le traitement. Le verre d'aplanation forme ainsi un plan de référence pour la focalisation du faisceau laser.

Le document US 2013/165911 divulgue un dispositif d'aplanation destiné à être positionné entre l'œil d'un patient et un système laser, pour maintenir en position l'œil et créer un plan de référence pour une opération de chirurgie ophtalmologique laser, ledit dispositif comprenant : un cône d'aplanation définissant un espace intérieur, une ouverture supérieure et une ouverture inférieure ; ledit cône d'aplanation comprenant une partie supérieure configurée pour être fixée sur une partie saillante d'un bloc optique de focalisation du système laser et une partie inférieure comportant des moyens d'aspiration ; l'ouverture inférieure étant délimitée par un rebord inférieur ; et un verre d'aplanation transparent au faisceau laser généré par le système laser.

### Problème technique

Selon les dispositifs d'aplanation connus, le verre d'aplanation est généralement fixé au cône d'aplanation par une couche de colle dont l'épaisseur n'est pas homogène sur toute la surface de la fixation. En outre, d'un dispositif d'aplanation à un autre, l'épaisseur n'est pas reproductible. Cette variation d'épaisseur peut induire des erreurs de positionnement du plan de référence formé par le verre d'aplanation par rapport au système de focalisation. Or il est essentiel que le verre d'aplanation soit positionné à une distance prédéterminée et précise du système de focalisation du système laser pour pouvoir focaliser le spot du faisceau à une profondeur choisie. Par conséquent, dans les solutions proposées, il est nécessaire de réaliser un contrôle de la distance entre la sortie optique du système optique de focalisation et le plan de référence formé par le verre d'aplanation pour chaque cône d'aplanation avant le début de l'opération. Ce contrôle conduit soit à changer le cône d'aplanation soit à une correction de cette distance, afin de compenser d'éventuelles imprécisions de profondeur de focalisation, rendant l'opération plus longue et complexe. Le dépôt de la colle peut également induire un défaut d'uniformité ou d'épaisseur sur le joint de colle qui introduirait un défaut d'horizontalité du verre d'aplanation. En outre, la colle peut subir un retrait lié à l'évaporation du solvant, pouvant fragiliser la fixation du verre d'aplanation. Enfin, des dépôts de colle peuvent également se retrouver sur la partie fonctionnelle du verre d'aplanation, dans le passage du laser, rendant ainsi la pièce formée par le cône d'aplanation et le verre d'aplanation non conforme pour l'utilisation.

Selon un autre inconvénient, le cône d'aplanation est généralement à usage unique, du fait de la fixation permanente du verre d'aplanation sur le cône. Il est donc nécessaire de remplacer à chaque opération l'ensemble le cône et le verre.

Selon encore un autre inconvénient, les dispositifs d'aplanation connus n'offrent généralement pas la possibilité de réajuster la distance entre le verre d'aplanation et le système de focalisation.

Un objectif de la présente invention est donc de proposer un dispositif d'aplanation qui permet de résoudre les problèmes techniques posés par les dispositifs de l'état de l'art antérieur, en mettant au point une structure particulièrement adaptée à être couplée à un système de chirurgie ophtalmologique laser, facile et pratique à utiliser, permettant de positionner avec une grande précision le verre d'aplanation sur l'œil, et également mobile dans la direction du faisceau laser pour pouvoir ajuster facilement la distance entre le verre d'aplanation et le système de focalisation.

### Exposé de l'invention

Afin de remédier aux inconvénients précités de l'état de la technique, la présente invention propose un dispositif d'aplanation destiné à être positionné entre l'œil d'un patient et un système laser, pour maintenir en position l'œil et créer un plan de référence pour une opération de chirurgie ophtalmologique laser, ledit dispositif comprenant :
- un cône d'aplanation définissant un espace intérieur, une ouverture supérieure et une ouverture inférieure ;
- ledit cône d'aplanation comprenant une partie supérieure configurée pour être fixée sur une partie saillante d'un bloc optique de focalisation du système laser et une partie inférieure comportant des moyens d'aspiration;
- l'ouverture inférieure étant délimitée par un rebord inférieur;
- un verre d'aplanation transparent au faisceau laser généré par le système laser étant positionné et maintenu par lesdits moyens d'aspiration contre le rebord inférieur de manière à venir fermer l'ouverture inférieure.

Selon un mode de réalisation de l'invention, les moyens d'aspiration comprennent au moins un canal d'aspiration s'étendant dans la paroi dudit cône d'aplanation entre une entrée d'aspiration et une pluralité d'orifices d'aspirations formés dans le rebord inférieur.

Selon un mode de réalisation de l'invention, le dispositif d'aplanation comprend en outre une bague de fixation configurée pour être montée fixement sur la partie inférieure saillante du bloc optique de focalisation, la partie supérieure dudit cône d'aplanation étant configurée pour être montée mobile en translation selon une direction parallèle à l'axe optique du bloc optique de focalisation sur ladite bague de fixation.

Selon un autre mode de réalisation de l'invention, le dispositif d'aplanation comprend en outre une bague d'orientation angulaire configurée pour être montée mobile en rotation dans un plan perpendiculaire à l'axe optique du bloc optique de focalisation sur ladite partie saillante, la partie supérieure dudit cône d'aplanation étant configurée pour être montée mobile en translation selon une direction parallèle à l'axe optique du bloc optique de focalisation sur ladite bague d'orientation angulaire.

Selon une forme de réalisation de l'invention, le cône d'aplanation comprend au moins un élément de maintien de caméra fixé sur la paroi extérieure du cône d'aplanation et au moins une ouverture latérale agencée par rapport au dit élément de maintien de manière à laisser passer un faisceau d'imagerie généré par ladite au moins une caméra pour visualiser une zone de découpe de l'œil située sous le verre d'aplanation.

De préférence, ledit élément de maintien est configuré de sorte que le faisceau d'imagerie généré par ladite caméra soit incliné d'un angle compris entre 30° et 50°, de préférence compris entre 45° et 47°, par rapport à un axe optique de symétrie du bloc de focalisation.

Les caractéristiques exposées dans les paragraphes suivants peuvent, optionnellement, être mises en oeuvre. Elles peuvent être mises en oeuvre indépendamment les unes des autres ou en combinaison les unes avec les autres :
- le verre d'aplanation comprend au moins une mire ou une réglette graduée réalisée en surface ou en volume ;
- le verre d'aplanation présente une géométrie plan-plan ou plan-concave ;
- le cône d'aplanation est réalisé dans un matériau métallique par fabrication additive.

Selon un autre mode de réalisation de l'invention, le cône d'aplanation comprend une bague de déplacement axial munie d'un filetage intérieur, conçue pour être en prise par vissage avec un filetage extérieur réalisé sur la paroi extérieure de la bague de fixation ou de la bague d'orientation, ladite bague de déplacement axial assurant dans une étape de vissage un déplacement axial dudit cône d'aplanation.

Selon un autre mode de réalisation de l'invention, le dispositif d'aplanation comprend en outre une bague de verrouillage configurée pour bloquer en position le cône d'aplanation.

Selon un exemple de réalisation, le cône d'aplanation comprend une partie inférieure fixée sur la partie supérieure, ladite partie inférieure présentant une forme de cône tronqué à l'envers.

L'invention propose également un ensemble d'aplanation pour maintenir en position l'œil d'un patient et créer un plan de référence pour un système laser pour une opération de chirurgie ophtalmologique laser, ledit ensemble comprenant :
- un dispositif d'aplanation tel que défini ci-dessus;
- un anneau de succion conçu pour venir en contact avec une surface de l'œil du patient ;
- une portion de ladite partie inférieure du cône d'aplanation étant configurée pour être insérée à l'intérieur de l'anneau pour positionner le verre d'aplanation à l'intérieur de l'anneau de succion de manière à venir en contact avec l'œil du patient.

L'invention a pour objet également un appareil de chirurgie ophtalmologique laser pour réaliser une découpe dans un tissu biologique oculaire, tel qu'une cornée ou un cristallin, comprenant :
- une unité laser adaptée pour délivrer un faisceau laser,
- un bloc optique de focalisation pour focaliser le faisceau laser en un point focal dans le tissu biologique oculaire;
- ledit bloc optique de focalisation comprenant une partie inférieure saillante s'étendant à partir d'une surface inférieure de l'unité laser,
- un dispositif d'aplanation tel que défini ci-dessus configuré pour être fixé sur la partie inférieure saillante du bloc optique de focalisation de manière à positionner le verre d'aplanation à une distance sensiblement proche de la distance de focalisation par rapport à la sortie optique du bloc optique de focalisation.

Selon un mode de réalisation de l'invention, l'appareil comprend en outre au moins une caméra configurée pour visualiser la zone de découpe, ladite au moins une caméra étant maintenue en position par l'un des éléments de maintien de caméra fixé sur la paroi extérieure du cône d'aplanation.

Selon un autre mode de réalisation de l'invention, l'appareil comprend en outre une caméra de centrage configurée pour le centrage de l'axe optique de symétrie du système de focalisation par rapport au tissu biologique, une extrémité de la caméra de centrage étant insérée dans le cône d'aplanation via une des deux ouvertures prévues du cône d'aplanation.

### Brève description des dessins

D'autres caractéristiques, détails et avantages de l'invention apparaîtront à la lecture de la description détaillée ci-après, et à l'analyse des dessins annexés, sur lesquels :
**Fig. 1**

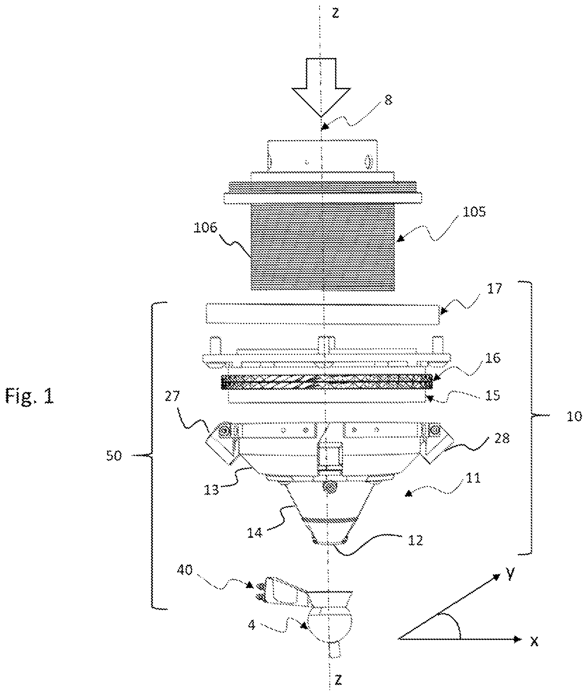

   [Fig. 1] représente une vue éclatée du dispositif d'aplanation selon un mode de réalisation de l'invention, positionné entre un bloc optique de focalisation et un anneau de succion placé au-dessus d'un oeil ;
**Fig. 2A**

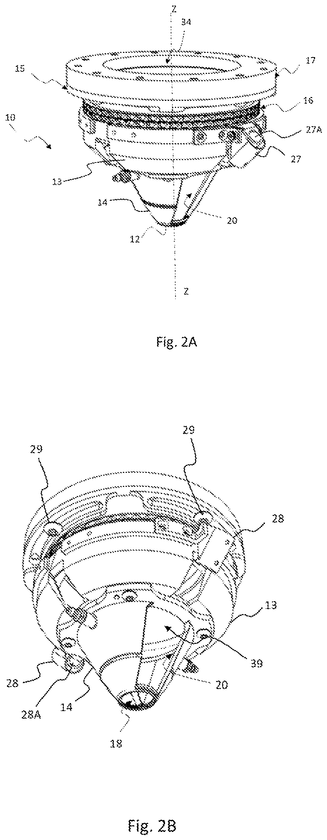

   [Fig. 2A] représente une vue en perspective du dispositif d'aplanation dans une configuration assemblée ;
**Fig. 2B**
   [Fig. 2B] représente une vue en perspective depuis une vue du dessous du dispositif de la figure 2A ;
**Fig. 3A**

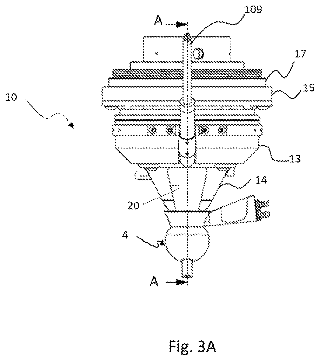

   [Fig. 3A] représente une vue de profil selon la vue de l'une des deux caméras de vision latérales du dispositif d'aplanation;
**Fig. 3B**

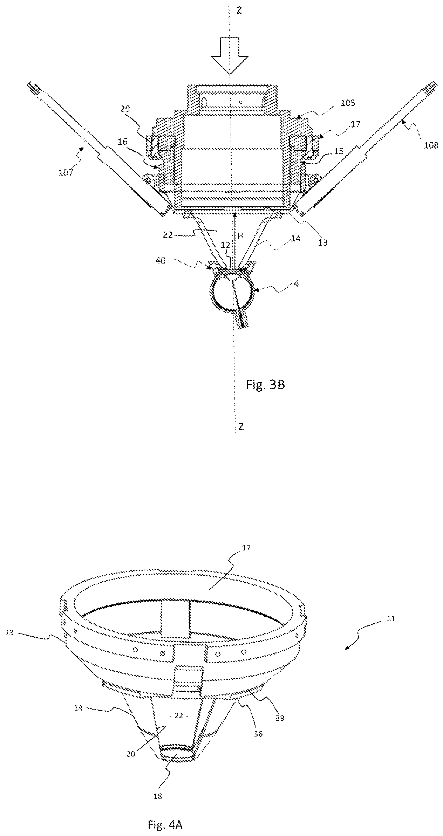

   [Fig. 3B] représente une vue en coupe du dispositif d'aplanation suivant la ligne A-A sur la figure 3A ;
**Fig. 4A**
   [Fig. 4A] représente une vue de profil en perspective du cône d'aplanation ;
**Fig. 4B**

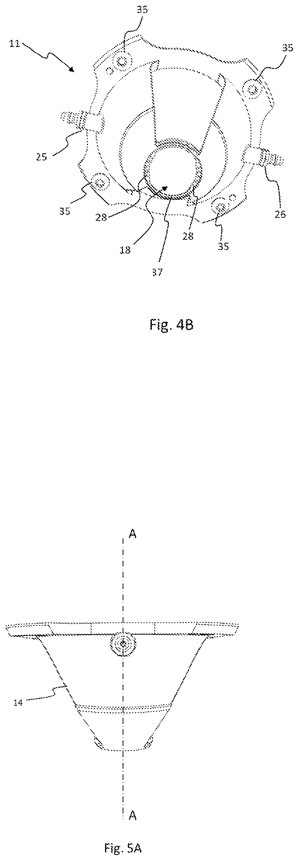

   [Fig. 4B] représente une vue en perspective du dessous du cône d'aplanation de la figure 4A sans le verre d'aplanation ;
**Fig. 5A**
   [Fig. 5A] représente une vue de profil du cône d'aplanation ;
**Fig. 5B**

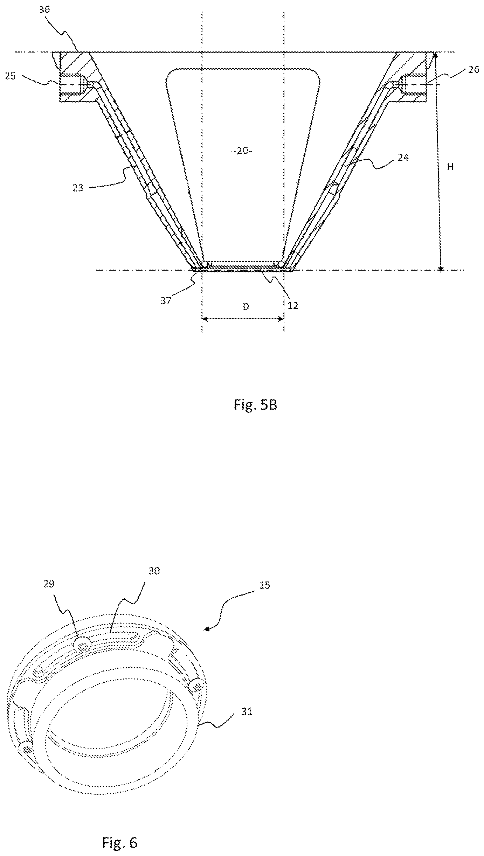

   [Fig. 5B] représente une vue en coupe du cône d'aplanation suivant la ligne A-A sur la figure 5A montrant les canaux d'aspiration ;
**Fig. 6**
   [Fig. 6] représente une vue en perspective de la bague d'orientation angulaire ;
**Fig. 7A**

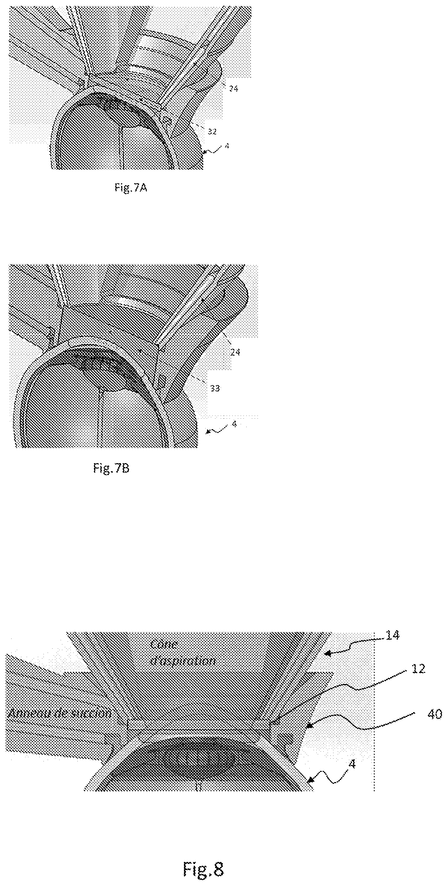

   [Fig. 7A] représente un exemple d'aplanation par un verre plan-plan en position d'appui contre la surface de la cornée de l'œil ;
**Fig. 7B**
   [Fig. 7B] représente un exemple d'aplanation par un verre plan-concave en position d'appui contre la surface de la cornée de l'œil ;
**Fig. 8**
   [Fig. 8] représente schématiquement une vue en coupe partielle de l'ensemble formé par un anneau de succion et une partie inférieure du cône d'aplanation et le verre d'aplanation appliqué sur la cornée de manière à conformer la surface de la cornée à la géométrie de la surface inférieure du verre d'aplanation qu'elle soit plane ou courbe ;
**Fig. 9**

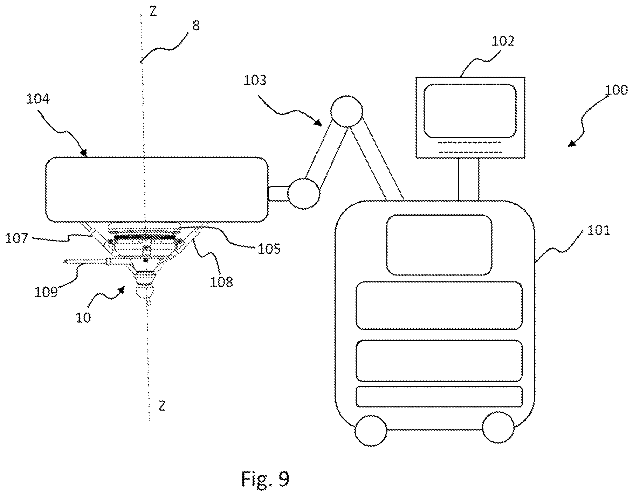

   [Fig. 9] représente schématiquement un appareil de chirurgie ophtalmologique équipé d'un dispositif d'aplanation selon un mode de réalisation de l'invention.
**Fig. 10A**

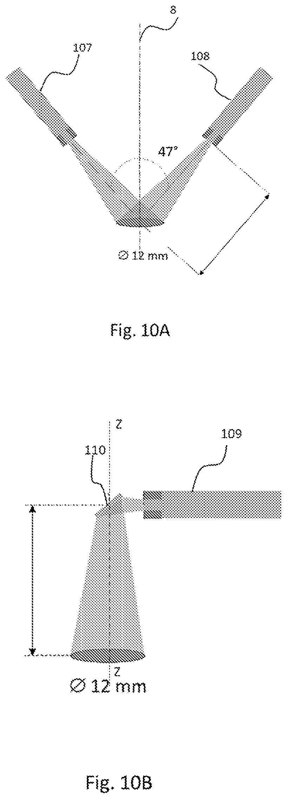

   [Fig. 10A] représente schématiquement une vue de face de l'agencement des deux caméras de vision latérales ;
**Fig. 10B**
   [Fig. 10B] représente schématiquement une vue de face de l'agencement de la caméra de centrage ;
Pour plus de clarté, les éléments similaires sont repérés par des signes de référence identiques sur l'ensemble des figures.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

La présente divulgation propose un dispositif d'aplanation qui permet, lors d'une opération de chirurgie ophtalmologique laser, en appliquant un verre d'aplanation au contact de l'œil à traiter, de stabiliser l'œil par rapport au point focal du faisceau laser. Ce dispositif permet également d'aplanir l'œil du patient au moyen du verre d'aplanation de manière à créer un plan de référence pour positionner le point focal du laser sur la zone à traiter de l'œil. Le dispositif d'aplanation est adapté pour être couplé d'une part à un anneau de succion positionné sur l'œil d'un patient et d'autre part à un système laser dédié à la chirurgie ophtalmologique laser. Le dispositif de la présente divulgation, par un mécanisme d'aspiration permet un positionnement et un maintien, précis, simple et reproductible du verre d'aplanation.

La figure 1 représente schématiquement un dispositif d'aplanation 10 selon un mode de réalisation. On a représenté ici schématiquement le dispositif d'aplanation dans un contexte d'utilisation pour une intervention chirurgicale de découpe de la cornée. Le dispositif est représenté ici entre un bloc optique de focalisation 105 et un anneau de succion 40 positionné sur l'œil 4. Le bloc optique de focalisation 105 est disposé sur le trajet optique d'un faisceau laser délivré par un système laser non illustré sur la figure et adapté pour focaliser le faisceau laser en un point focal dans l'épaisseur de la cornée. Le faisceau laser délivré par le système laser est représenté ici par une flèche en direction du bloc optique de focalisation 105. Un axe optique de symétrie 8 passant par le centre de la cornée et étant perpendiculaire à la surface de la cornée s'étend suivant une direction Z-Z orthogonale à un plan (XY). La cornée lorsqu'elle est aplanie par le verre d'aplanation s'étend sensiblement dans le plan (XY). Pendant l'opération de chirurgie ophtalmologique, le faisceau laser incident est focalisé à différentes profondeurs en volume dans la cornée selon une direction parallèle à l'axe Z avec un angle d'incidence normal.

Selon un mode de réalisation de l'invention, le dispositif d'aplanation 10 comprend un cône d'aplanation 11 et un verre d'aplanation 12 maintenu par aspiration sur une partie inférieure du cône d'aplanation 11, une bague d'orientation angulaire 15 et une bague de fixation 17 qui est montée fixement par vissage sur une partie saillante 106 du bloc optique de focalisation 105 lorsque le dispositif d'aplanation est couplé au système laser.

De préférence, le verre d'aplanation est en matériau transparent dans le visible, dont la surface est de qualité optique, chimiquement inerte, d'une grande stabilité dimensionnelle.

Les figures 2A et 2B illustrent le dispositif dans une configuration assemblée. Le cône d'aplanation creux 11 reçoit une partie inférieure de la bague d'orientation angulaire 15 qui est elle-même fixée via des vis de fixations sur la bague de fixation 17. Dans la configuration où le dispositif d'aplanation est couplé au système laser pour une opération de chirurgie ophtalmologique, la bague de fixation 17 est montée fixement sur la partie saillante 106 du bloc optique de focalisation 105. Lorsque toutes les pièces du dispositif sont assemblées, le dispositif est défini par un espace intérieur 39, une ouverture supérieure 34 et une ouverture inférieure 18 fermée par le verre d'aplanation 12 qui est transparent à la longueur d'onde du faisceau laser. L'axe de symétrie du dispositif d'aplanation 10 est sensiblement confondu avec l'axe optique de symétrie 8 de la cornée et du bloc optique de focalisation 105.

Selon un mode de réalisation particulièrement avantageux, le dispositif d'aplanation comprend en outre deux éléments de maintien de caméra 27, 28 fixés sur la paroi extérieure du cône d'aplanation et deux ouvertures latérales 20 réalisées dans la paroi d'une partie inférieure du cône d'aplanation 13.

L'élément de maintien 27, 28 comprend chacun un logement tubulaire 27A, 28A configuré pour recevoir une caméra de vision 107, 108, dites caméras de vision latérale. Comme illustré sur les figures 10A et 10B, les caméras sont orientées de manière à générer un faisceau d'imagerie incliné d'un angle compris entre 30° et 50°, de préférence compris entre 45° et 47, par rapport à l'axe optique de symétrie du bloc de focalisation. Les ouvertures 20 sont de ce fait agencées par rapport aux éléments de maintien de manière à laisser passer le faisceau d'imagerie généré par la caméra pour visualiser une zone de découpe de l'œil située sous le verre d'aplanation. Les ouvertures 20 sont situées à l'aplomb des éléments de maintien 27, 28.

Les caméras 107, 108 permettent au chirurgien de visualiser sur un écran d'affichage la cornée pendant l'opération. Grâce à la présence des ouvertures latérales 20, il est possible d'introduire une caméra de centrage 109 (figure 9) à l'intérieur du cône d'aplanation dont le cône de vision est centré par rapport à l'axe optique de la cornée de manière à bien positionner l'appareil de chirurgie au-dessus de l'œil du patient.

La caméra de centrage 109 est mobile selon une direction transversale entre une première position où elle est dans l'espace intérieur du cône d'aplanation 11, agencée entre le système de focalisation et le tissu pour réaliser le centrage et une seconde position où elle est retirée avant le début de l'opération.

Lorsque le cône d'aplanation 11 est monté et positionné sur la bague d'orientation angulaire 15, il est nécessaire de s'assurer que l'une des ouvertures 20 soit en regard de la caméra de centrage 109. Pour cela et en référence à la figure 6, la bague d'orientation 15 est munie par exemple de quatre trous oblong 30 dans lesquels sont agencés des vis de fixation 29. Après avoir ajusté la distance ***H*** entre la sortie optique du bloc de focalisation et le verre d'aplanation en tournant le cône d'aplanation 11 sur la bague d'orientation 15 pour générer un déplacement axial suivant l'axe Z-Z, il est possible de tourner la bague d'orientation angulaire 15 dans le plan (XY) en déplaçant les vis de fixation 29 dans les trous oblongs 30 de manière à placer l'une des ouvertures 20 en regard de la caméra de centrage. La rotation dans le plan (XY) n'induit pas de déplacement axial suivant l'axe Z-Z. A la fin de cette rotation, les vis de fixation 29 sont fixés sur la bague de fixation 17.

En référence aux figures 3 et 4, le cône d'aplanation 11 est un corps creux défini par un espace intérieur 22, une ouverture supérieure 17 et une ouverture inférieure 18. L'ouverture inférieure 18 est centrée par rapport à l'ouverture supérieure 17. Le cône d'aplanation 11 comprend une partie supérieure 13 et une partie inférieure 14. La partie supérieure 13 se présente sous la forme d'une bague ayant la forme d'un cône tronqué à l'envers défini par un espace intérieur sensiblement cylindrique. Elle est pourvue sur sa paroi interne d'un filetage intérieur. La dimension de l'espace intérieur est adaptée pour recevoir une partie inférieure de la bague d'orientation 15 qui présente une forme cylindrique. Le filetage intérieur et le filetage extérieur réalisé sur la paroi externe de la bague d'orientation coopèrent ensemble pour déplacer le corps d'aplanation 11 suivant l'axe Z-Z sur la bague d'orientation 15 afin d'ajuster la distance ***H*** entre la sortie optique du bloc optique de focalisation 105 et le verre d'aplanation 12. La distance ***H*** correspond sensiblement à la distance de travail du bloc optique de focalisation. Elle est comprise généralement entre 20 mm et 70 mm, de préférence comprise entre 30 et 50 mm. Pour la suite de la description, la partie supérieure 13 du cône d'aplanation est nommée bague de déplacement axial.

En référence aux figures 5A et 5B, la partie inférieure 14 présente également la forme d'un cône tronqué à l'envers. Elle comprend un rebord supérieur 36 et un rebord inférieur 37 délimitant l'ouverture inférieur 18. Le rebord inférieur 37 forme une surface d'aspiration pour maintenir le verre d'aplanation 12 qui vient fermer l'ouverture inférieure 18. Le verre d'aplanation 12 présente une forme sensiblement circulaire et réalisée dans un matériau transparent à la longueur d'onde du faisceau laser délivré par le système laser. Le diamètre du verre d'aplanation est sensiblement égal au diamètre de la base du cône tronqué 14. Le diamètre de l'ouverture inférieure ***D*** forme le diamètre du champ de travail du faisceau laser qui est environ 10 mm au niveau de l'œil du patient.

La partie inférieure 14 comprend des moyens d'aspiration pour maintenir le verre d'aplanation contre le rebord inférieur. Selon un exemple de réalisation illustré sur la figure 5B, ils comprennent deux canaux d'aspiration 23, 24 aménagés dans la paroi de la partie inférieure du corps d'aspiration. Pour la suite de la description, on désigne la partie inférieure par le terme « cône d'aspiration ». Chaque canal 23, 24 s'étend entre une entrée d'aspiration 25, 26 et une sortie d'aspiration située dans le rebord inférieur 37. Selon un exemple de réalisation et en référence à la figure 4B, la sortie d'aspiration est formée d'une pluralité d'orifices d'aspiration 28 situés dans le rebord inférieur 37. Les deux principaux canaux 23, 24 sont donc divisés en une pluralité de sous-canaux qui sont connectés aux orifices d'aspiration 28. Les deux entrées 25, 26 sont pourvues chacune d'un connecteur adapté pour être connecté à un moyen de génération de vide. Lorsque le verre d'aplanation est placé sur le rebord inférieur, un vide est appliqué aux deux canaux annulaires via les deux entrées et génère une force d'aspiration qui plaque solidement le verre d'aplanation contre le rebord inférieur.

En référence à la figure 5B, lorsque les deux parties sont assemblées ensemble, le rebord supérieur 36 du cône d'aspiration 14 est en appui contre la base 39 du cône tronqué de la partie supérieure 13. Les deux parties sont fixées ensemble par quatre vis 35.

Selon un mode de réalisation de l'invention et en référence à la figure 1, le dispositif d'aplanation comprend en outre deux bagues de verrouillage 16 pour bloquer en position le corps d'aplanation 11 sur la bague d'orientation angulaire 15.

Selon un mode de réalisation de l'invention, le dispositif d'aplanation est réalisé dans un matériau métallique tel que Cr-Co par une technique de fabrication additive, dite fusion laser sélective ou lit de poudre, pour une question de faisabilité technique d'une part, et de prix d'autre part. L'ébauche réalisée par fabrication additive est ensuite rectifiée par fraisage mécanique pour imposer une hauteur déterminée, et polie par microbillage, micro-sablage ou polissage manuel.

En référence à la figure 7A et à la figure 7B, lorsque le dispositif d'aplanation est positionné dans l'anneau de succion 40 dans une configuration prête pour l'opération, le verre d'aplanation est en appui contre la cornée de l'œil du patient pour aplanir la surface de l'œil afin de créer un plan de référence pour les différentes opérations laser à réaliser dans la cornée. Selon un exemple de réalisation illustrée sur la figure 7A, le verre d'aplanation 32, dit verre plan-plan est plat sur ses deux surfaces. Ce verre est utilisé pour une grande majorité d'opérations, telle qu'une découpe en surface ou proche de la face supérieure de la cornée (exemples : LASIK ou KLA). Il présente une épaisseur d'environ 1 mm par exemple et de diamètre 14 mm. Selon un autre exemple de réalisation illustré sur la figure 7B, il peut avoir une surface supérieure plate et une surface inférieure concave disposée en regard de la cornée. Le rayon de courbure de la surface concave est légèrement supérieur au rayon de courbure moyen de la cornée. Ce verre nommé plan-concave 33 est utilisé pour des opérations plus spécifiques, telles qu'une découpe en profondeur afin d'éviter la formation de plis dans le tissu cornée (exemples : ablation lenticulaires, poches intra stromales, KT ou KLP). Il présente un diamètre de 15 mm et une épaisseur au centre d'environ 2 mm.

Selon un mode de réalisation, le verre d'aplanation est muni d'une mire et/ou une réglette gravée en surface ou en volume pour aider un positionnement précis du cône d'aplanation par rapport au centre de l'œil du patient, et pour visualiser le diamètre d'aplanation de l'œil. Des caractères alphanumériques peuvent aussi être ajoutés au marquage si besoin. Les gravures peuvent être réalisées par laser pico ou femtoseconde.

Pour placer précisément le verre d'aplanation par rapport au point focal du laser, la dimension en hauteur du cône d'aspiration sera importante à respecter. Grâce à la partie supérieure 13 du corps d'aplanation qui est mobile en translation suivant l'axe Z-Z, il est possible d'affiner la distance entre la sortie optique du bloc optique de focalisation et le verre d'aplanation pour qu'elle corresponde sensiblement à la distance de travail du bloc optique de focalisation. En pratique, il faut ajuster la hauteur du cône d'aspiration de manière à positionner le point focal du faisceau laser juste en-dessous du verre d'aplanation. La tolérance sur le positionnement du verre d'aplanation par rapport au bloc optique de focalisation est de ± 10 µm.

Comme illustré sur la figure 1, le bloc optique de focalisation 105 présente généralement une forme sensiblement cylindrique dont l'axe est parallèle à l'axe Z-Z et est centré sur l'axe optique de symétrie de la cornée 8. Le bloc optique de focalisation est constitué d'un assemblage de lentilles de manière à focaliser le faisceau laser dans l'épaisseur de la cornée pour obtenir des points d'impact de taille constante à l'échelle de quelques microns sur un champ de travail de diamètre d'au moins 10 mm. Lorsque le bloc optique de focalisation est intégré dans une unité laser, il présente une partie saillante 106 par rapport à la surface de l'unité laser. La paroi extérieure de la partie saillante 106 du bloc optique est munie d'un filetage extérieur permettant à la bague de fixation 17 qui est munie d'un filetage intérieur complémentaire d'y fixer par vissage.

Selon un mode de réalisation et en référence à la figure 8, le dispositif d'aplanation est couplé à l'œil par l'intermédiaire d'un anneau de succion 40 qui a pour fonction de guider le cône d'aplanation lors du positionnement du verre d'aplanation 12 sur l'œil. L'anneau de succion est positionné et appliqué sur l'œil du patient au préalable par le praticien. L'anneau de succion 40 comprend des moyens d'aspiration qui permettent de le maintenir en position à une partie cornéenne de l'œil du patient. Pour coupler le dispositif d'aplanation à l'anneau de succion, une portion de la partie inférieure du cône d'aplanation 11 du dispositif d'aplanation 10 est adaptée dimensionnellement et géométriquement pour être reçue dans l'anneau de succion 40 de manière à positionner le verre d'aplanation 12 dans l'anneau de succion afin d'être mis en contact contre l'œil du patient.

En référence à la figure 9, la présente divulgation concerne également un appareil de chirurgie ophtalmologique 100 pour réaliser une découpe dans un tissu biologique oculaire, tel qu'une cornée ou un cristallin. Il comprend une unité laser 104 adaptée pour délivrer un faisceau laser. Cette unité comprend un bloc de focalisation pour focaliser le faisceau laser en un point focal dans le tissu biologique oculaire et un dispositif d'aplanation 10 tel que décrit ci-dessus.

L'unité laser 104 comprend également un système optique de déplacement du faisceau laser. L'unité laser est adaptée pour être montée à l'extrémité d'un bras articulé 103. L'autre extrémité du bras est montée sur une baie électrotechnique mobile 101. Une partie des éléments de l'équipement est logée dans la baie électrotechnique mobile 101. Le bras articulé auto-équilibré permet de positionner l'unité laser au-dessus de l'œil du patient.

Le bloc optique de focalisation 105 illustré plus en détail sur la figure 1 comprend une partie inférieure saillante munie sur sa paroi externe un filetage et qui s'étend à partir d'une surface inférieure de l'unité laser 104. Le dispositif d'aplanation est fixé sur la partie saillante du bloc optique de focalisation via la bague de fixation 17.

Selon un mode de réalisation de l'invention, l'appareil comprend deux caméras 107, 108 configurées pour visualiser la zone de découpe, maintenue en position par des éléments de maintien de caméra 27, 28 fixés sur la paroi extérieure du corps d'aplanation 11. Comme l'illustre la figure 3B, les deux caméras sont orientées de manière à diriger les deux faisceaux d'imagerie vers l'intérieur du cône d'aspiration 14 via des ouvertures correspondantes 20.

Comme l'illustre la figure 10A, les deux caméras de vision latérale 107, 108, dotées d'un anneau d'éclairage, positionnées de part et d'autre de l'axe de symétrie optique 8 du bloc de focalisation permettent de visualiser et illuminer l'œil pendant l'opération. Le diamètre de la zone d'image qui est d'environ 12 mm est supérieur à la zone de découpe où se déplace le spot laser. Les deux caméras permettent d'observer la zone de découpe et de suivre le processus de découpe. Les deux caméras sont équipées d'un anneau d'éclairage et d'un autofocus. Les deux caméras sont positionnées entre le bloc optique de focalisation et la cornée de sorte que le faisceau d'imagerie soit orienté d'un angle compris entre 45° et 47° par rapport à un axe optique de symétrie du système de focalisation. Ces valeurs permettent la visualisation de l'œil sur un champ de 10,5 mm tout en prenant en compte l'encombrement des optiques et mécaniques du dispositif, des caméras et du dispositif d'aplanation.

Comme l'illustre la figure 10B, l'appareil comprend en outre une caméra de centrage 109 configurée pour le centrage de l'axe optique du bloc optique focalisation par rapport au tissu biologique, une extrémité de la caméra de centrage étant insérée dans le cône d'aspiration 14 via une des deux ouvertures latérales 20.

La caméra de centrage 109 est une caméra amovible qui est agencée entre le bloc optique de focalisation et l'œil. Elle génère un cône de vision qui est renvoyé à 90° par un miroir 110 de manière à générer un cône de vision centré sur l'axe optique 8 du système de focalisation, permettant ainsi de positionner l'appareil au-dessus de l'œil du patient. Avantageusement, la caméra est équipée d'un anneau d'éclairage générant un spot annulaire d'alignement permettant ainsi au patient de fixer un point lumineux centré sur l'axe optique de symétrie du système de focalisation. Elle est également équipée d'un autofocus. La caméra de centrage 109 est retirée du corps d'aplanation pour démarrer l'opération.

La présente invention permet de disposer d'un dispositif d'aplanation efficace et simple d'utilisation pour coupler l'œil du patient avec un système laser pour réaliser des découpes de grande qualité dans un tissu biologique oculaire, tel que la cornée ou le cristallin. Un des avantages du dispositif d'aplanation de la présente divulgation consiste à utiliser un mécanisme d'aspiration intégré dans la paroi du cône d'aplanation pour maintenir fixement le verre d'aplanation contre une surface du cône d'aplanation. Cela permet de s'affranchir de la variation de l'épaisseur de la colle utilisée dans les dispositifs connus qui introduit une variation de distance entre la sortie optique du bloc de focalisation et le verre d'aplanation dans le plan (XY). L'absence de la couche de colle entre la surface d'appui pour le positionnement du verre et le verre d'aplanation permet d'éviter un mauvais positionnement du verre d'aplanation par rapport au point focal du laser qui pourrait impacter la qualité de la découpe. Grâce à la précision et la reproductibilité du positionnement du verre d'aplanation, il n'est plus nécessaire de procéder à une étape de calibration avant le début de l'opération pour connaître la position du point focal formé dans le tissu oculaire. La tolérance sur le positionnement du verre d'aplanation par rapport au système de focalisation est de ± 10 µm.

Une combinaison spécifique de plusieurs pièces mécaniques de fonctions différentes offre en outre la possibilité d'ajuster la distance entre le verre d'aplanation et le bloc optique de focalisation, afin de rendre le processus de couplage le plus précis possible tout en simplifiant le processus de couplage entre le dispositif d'aplanation et le système laser.

Grâce au mécanisme d'aspiration qui permet d'éviter une fixation permanente du verre sur le cône, ce dernier est réutilisable après stérilisation, seul le verre est consommable.

Le dispositif d'aplanation de la présente invention permet enfin d'intégrer facilement des caméras de vision et d'introduire une caméra de vision à l'intérieur du cône d'aspiration.

Le dispositif de la présente invention présente un coût de fabrication relativement faible, car il est réalisé dans un matériau métallique, par exemple en Cr-Co permettant une fabrication par fabrication additive.

### Application industrielle

Le dispositif d'aplanation de l'invention peut être utilisé en couplage avec différents systèmes laser tel que laser femtosecondes ou picosecondes pour les différentes opérations laser à réaliser dans la cornée et la chirurgie réfractive.

## Revendications

1. Dispositif d'aplanation (10) destiné à être positionné entre l'œil (4) d'un patient et un système laser, pour maintenir en position l'œil et créer un plan de référence pour une opération de chirurgie ophtalmologique laser, ledit dispositif comprenant :
- un cône d'aplanation (11) définissant un espace intérieur, une ouverture supérieure et une ouverture inférieure ;
- ledit cône d'aplanation (11) comprenant une partie supérieure (13) configurée pour être fixée sur une partie saillante (106) d'un bloc optique de focalisation (105) du système laser et une partie inférieure (14) comportant des moyens d'aspiration;
- l'ouverture inférieure étant délimitée par un rebord inférieur;
- un verre d'aplanation (12) transparent au faisceau laser généré par le système laser étant positionné et maintenu par lesdits moyens d'aspiration contre le rebord inférieur de manière à venir fermer l'ouverture inférieure.

2. Dispositif d'aplanation selon la revendication 1, dans lequel les moyens d'aspiration comprennent au moins un canal d'aspiration (23, 24) s'étendant dans la paroi dudit cône d'aplanation (11) entre une entrée d'aspiration (25, 26) et une pluralité d'orifices d'aspirations (28) formés dans le rebord inférieur (37).

3. Dispositif d'aplanation selon la revendication 1 ou 2, comprenant en outre une bague de fixation (17) configurée pour être montée fixement sur la partie saillante (106) du bloc optique de focalisation (105), la partie supérieure (13) dudit cône d'aplanation étant configurée pour être montée mobile en translation selon une direction parallèle à un axe optique (8) du bloc optique de focalisation sur ladite bague de fixation (17).

4. Dispositif d'aplanation selon la revendication 3, comprenant en outre une bague d'orientation angulaire (15) configurée pour être montée mobile en rotation dans un plan (XY) perpendiculaire à l'axe optique (8) du bloc optique de focalisation sur ladite partie saillante, la partie supérieure (13) dudit cône d'aplanation étant configurée pour être montée mobile en translation selon une direction parallèle à l'axe optique du bloc optique de focalisation sur ladite bague d'orientation angulaire (15).

5. Dispositif d'aplanation selon l'une des revendications précédentes, dans lequel le cône d'aplanation (11) comprend au moins un élément de maintien de caméra (27, 28) fixé sur la paroi extérieure du cône d'aplanation (11) et au moins une ouverture latérale (20) agencée par rapport au dit élément de maintien de manière à laisser passer un faisceau d'imagerie généré par ladite au moins une caméra pour visualiser une zone de découpe de l'œil située sous le verre d'aplanation (12).

6. Dispositif d'aplanation selon la revendication 5, dans lequel ledit élément de maintien (27, 28) est configuré de sorte que le faisceau d'imagerie généré par ladite caméra soit inclinée d'un angle compris entre 30° et 50°, de préférence compris entre 45° et 47°, par rapport à l'axe optique (8) du bloc optique de focalisation.

7. Dispositif d'aplanation selon l'une des revendications précédentes, dans lequel le verre d'aplanation (12) comprend au moins une mire ou une réglette graduée réalisée en surface ou en volume.

8. Dispositif d'aplanation selon l'une des revendications précédentes, dans lequel le verre d'aplanation présente une géométrie plan-plan (32) ou plan-concave (33).

9. Dispositif d'aplanation selon l'une des revendications 3 à 8, dans lequel la partie supérieure dudit cône d'aplanation (11) comprend une bague de déplacement axial (13) munie d'un filetage intérieur, conçue pour être en prise par vissage avec un filetage extérieur réalisé sur la paroi extérieure de la bague de fixation (17) ou de la bague d'orientation (15), ladite bague de déplacement axial (13) assurant dans une étape de vissage un déplacement axial dudit cône d'aplanation (11) selon une direction parallèle à l'axe optique (8) du bloc optique de focalisation.

10. Dispositif d'aplanation selon l'une des revendications 3 à 9, comprenant en outre une bague de verrouillage (16) configurée pour bloquer en position le cône d'aplanation (11).

11. Dispositif d'aplanation selon l'une des revendications 1 à 10, dans lequel le cône d'aplanation est réalisé dans un matériau métallique par fabrication additive.

12. Dispositif d'aplanation selon l'une des revendications 1 à 11, dans lequel le cône d'aplanation comprend une partie inférieure (14) fixée sur la partie supérieure (13), ladite partie inférieure présentant une forme de cône tronqué à l'envers.

13. Ensemble d'aplanation (50) pour maintenir en position l'œil d'un patient et créer un plan de référence pour un système laser pour une opération de chirurgie ophtalmologique laser, ledit ensemble comprenant :
- un dispositif d'aplanation (10) selon l'une des revendications 1 à 12 ;
- un anneau de succion (40) conçu pour venir en contact avec une surface de l'œil du patient ;
- une portion de ladite partie inférieure du cône d'aplanation étant configurée pour être insérée à l'intérieur de l'anneau de succion pour positionner le verre d'aplanation à l'intérieur de l'anneau de succion.

14. Appareil de chirurgie ophtalmologique (100) pour réaliser une découpe dans un tissu biologique oculaire, tel qu'une cornée ou un cristallin, comprenant :
- une unité laser (104) adaptée pour délivrer un faisceau laser,
- un bloc optique de focalisation (105) pour focaliser le faisceau laser en un point focal dans le tissu biologique oculaire;
- ledit bloc optique de focalisation comprenant une partie inférieure saillante (106) s'étendant à partir d'une surface inférieure de l'unité laser,
- un dispositif d'aplanation (10) selon l'une des revendications 1 à 12 configuré pour être fixé sur la partie inférieure saillante du bloc optique de focalisation de manière à positionner le verre d'aplanation à une distance sensiblement proche de la distance de focalisation par rapport à la sortie optique du bloc optique de focalisation.

15. Appareil selon la revendication 14, comprenant en en outre au moins une caméra (107, 108) configurée pour visualiser la zone de découpe, ladite au moins une caméra étant maintenue en position sur la paroi extérieure du cône d'aplanation.

16. Appareil selon l'une des revendications 14 ou 15, comprenant en outre une caméra de centrage (109) configurée pour le centrage de l'axe optique de symétrie du système de focalisation par rapport au tissu biologique, une extrémité de la caméra de centrage étant insérée dans le cône d'aplanation via une des deux ouvertures prévues du cône d'aplanation.

## Patentansprüche

1. Applanationsvorrichtung (10) zur Positionierung zwischen dem Auge (4) eines Patienten und einem Lasersystem, um das Auge in Position zu halten und eine Referenzebene für eine ophthalmologische Laseroperation zu schaffen, wobei die Vorrichtung umfasst:
- einen Applanationskonus (11), der einen Innenraum, eine obere Öffnung und eine untere Öffnung definiert,
- wobei der Applanationskonus (11) einen oberen Teil (13) umfasst, der dazu ausgebildet ist, an einem vorstehenden Teil (106) eines fokussierenden optischen Blocks (105) des Lasersystems befestigt zu werden, und einen unteren Teil (14) mit einer Saugeinrichtung umfasst,
- wobei die untere Öffnung durch einen unteren Rand begrenzt ist,
- ein Applanationsglas (12), das für den vom Lasersystem erzeugten Laserstrahl transparent ist und von den Ansaugmitteln gegen den unteren Rand positioniert und gehalten wird, so dass es die untere Öffnung verschließt.

2. Applanationsvorrichtung nach Anspruch 1, wobei die Saugmittel wenigstens einen Saugkanal (23, 24) umfassen, der sich in der Wand des Applanationskonus (11) zwischen einem Saugeinlass (25, 26) und einer Vielzahl von Saugöffnungen (28), die in der unteren Kante (37) ausgebildet sind, erstreckt.

3. Applanationsvorrichtung nach Anspruch 1 oder 2, ferner umfassend einen Befestigungsring (17), der dazu ausgebildet ist, fest an dem vorstehenden Teil (106) des fokussierenden optischen Blocks (105) angebracht zu werden, wobei der obere Teil (13) des Applanationskonus dazu ausgebildet ist, translationsbeweglich in einer Richtung parallel zu einer optischen Achse (8) des fokussierenden optischen Blocks an dem Befestigungsring (17) angebracht zu werden.

4. Applanationsvorrichtung nach Anspruch 3, welche ferner einen Winkelorientierungsring (15) umfasst, der dazu ausgebildet ist, in einer Ebene (XY) senkrecht zur optischen Achse (8) des fokussierenden optischen Blocks an dem vorstehenden Teil drehbar angebracht zu werden, wobei der obere Teil (13) des Applanationskonus dazu ausgebildet ist, in einer Richtung parallel zur optischen Achse des fokussierenden optischen Blocks an dem Winkelorientierungsring (15) translatorisch beweglich angebracht zu werden.

5. Applanationsvorrichtung nach einem der vorhergehenden Ansprüche, wobei der Applanationskonus (11) wenigstens ein an der Außenwand des Applanationskonus (11) befestigtes Kamerahalteelement (27, 28) und wenigstens eine seitliche Öffnung (20) umfasst, die in Bezug auf das Halteelement so angeordnet ist, dass sie einen von der wenigstens einen Kamera erzeugten Abbildungsstrahl durchlässt, um einen unter dem Applanationsglas (12) befindlichen Schnittbereich des Auges zu visualisieren.

6. Applanationsvorrichtung nach Anspruch 5, wobei das Halteelement (27, 28) derart ausgebildet ist, dass der von der Kamera erzeugte Abbildungsstrahl um einen Winkel zwischen 30° und 50°, bevorzugt zwischen 45° und 47°, bezüglich der optischen Achse (8) des fokussierenden optischen Blocks geneigt ist.

7. Applanationsvorrichtung nach einem der vorhergehenden Ansprüche, wobei das Applanationsglas (12) wenigstens eine Messlatte oder einen Maßstab umfasst, die bzw. der als Fläche oder Volumen ausgeführt ist.

8. Applanationsvorrichtung nach einem der vorhergehenden Ansprüche, wobei das Applanationsglas eine planar-planare (32) oder planar-konkave (33) Geometrie aufweist.

9. Applanationsvorrichtung nach einem der Ansprüche 3 bis 8, wobei der obere Teil des Applanationskonus (11) einen Axialverschiebungsring (13) mit einem Innengewinde umfasst, der derart ausgebildet ist, dass er mit einem Außengewinde, das an der Außenwand des Befestigungsrings (17) oder des Ausrichtungsrings (15) ausgebildet ist, in Schraubeingriff kommt, wobei der Axialverschiebungsring (13) in einem Schraubschritt eine Axialverschiebung des Applanationskonus (11) in einer Richtung parallel zur optischen Achse (8) des fokussierenden optischen Blocks gewährleistet.

10. Applanationsvorrichtung nach einem der Ansprüche 3 bis 9, welche ferner einen Verriegelungsring (16) umfasst, der dazu ausgebildet ist, den Applanationskonus (11) in seiner Position zu blockieren.

11. Applanationsvorrichtung nach einem der Ansprüche 1 bis 10, wobei der Applanationskonus durch additive Fertigung aus einem metallischen Material hergestellt ist.

12. Applanationsvorrichtung nach einem der Ansprüche 1 bis 11, wobei der Applanationskonus einen unteren Teil (14) aufweist, der an dem oberen Teil (13) befestigt ist, wobei der untere Teil die Form eines umgekehrten Konusstumpfes aufweist.

13. Applanationsvorrichtung (50), um das Auge eines Patienten in Position zu halten und eine Referenzebene für ein Lasersystem für eine ophthalmologische Laseroperation zu schaffen, wobei die Anordnung umfasst:
- eine Applanationsvorrichtung (10) nach einem der Ansprüche 1 bis 12,
- einen Saugring (40), der dazu ausgebildet ist, mit einer Oberfläche des Auges des Patienten in Kontakt zu kommen,
- wobei ein Abschnitt des unteren Teils des Applanationskonuss derart ausgebildet ist, dass er in das Innere des Saugrings eingeführt werden kann, um das Applanationsglas innerhalb des Saugrings zu positionieren.

14. Augenchirurgische Vorrichtung (100) zum Durchführen eines Schnitts in einem biologischen Augengewebe, wie einer Hornhaut oder einer Linse, umfassend:
- eine Lasereinheit (104), die dazu ausgebildet ist, einen Laserstrahl abzugeben,
- einen fokussierenden optischen Block (105) zum Fokussieren des Laserstrahls auf einen Brennpunkt in dem biologischen Augengewebe,
- wobei der fokussierende optische Block einen vorstehenden unteren Teil (106) umfasst, der sich von einer unteren Fläche der Lasereinheit aus erstreckt,
- eine Applanationsvorrichtung (10) nach einem der Ansprüche 1 bis 12, die dazu ausgebildet ist, an dem vorstehenden unteren Teil des fokussierenden optischen Blocks befestigt zu werden, um das Applanationsglas in einem Abstand im Wesentlichen nahe der Fokussierungsdistanz zu dem optischen Ausgang des fokussierenden optischen Blocks zu positionieren.

15. Vorrichtung nach Anspruch 14, ferner umfassend wenigstens eine Kamera (107, 108), die dazu ausgebildet ist, den Schnittbereich zu visualisieren, wobei die wenigstens eine Kamera an der Außenwand des Applanationskonus in Position gehalten ist.

16. Vorrichtung nach einem der Ansprüche 14 oder 15, ferner umfassend eine Zentrierkamera (109), die zum Zentrieren der optischen Symmetrieachse des Fokussierungssystems in Bezug auf das biologische Gewebe ausgebildet ist, wobei ein Ende der Zentrierkamera über eine der beiden vorgesehenen Öffnungen des Aplanationskonus in den Applanationskonus eingeführt ist.

## Claims

1. An applanation device (10) configured to be positioned between the eye (4) of a patient and a laser system, to hold the eye in position and create a reference plane for a laser ophthalmological surgery operation, said device comprising:
- an applanation cone (11) defining an internal space, a top aperture and a bottom aperture;
- said applanation cone (11) comprising an upper part (13) configured to be fixed onto a protruding part (106) of a focusing optical block (105) of the laser system and a lower part (14) comprising suction means;
- the bottom aperture being delimited by a bottom rim;
- an applanation glass (12) that is transparent to the laser beam generated by the laser system being positioned and held by said suction means against the bottom rim so as to close the bottom aperture.

2. The applanation device of claim 1, wherein the suction means comprise at least one suction channel (23, 24) extending in the wall of said suction cone (11) between a suction input (25, 26) and a plurality of suction orifices (28) formed in the bottom rim (37).

3. The applanation device of claim 1 or 2, further comprising a fixing ring (17) configured to be securely mounted on the protruding part (106) of the focusing optical block (105), the upper part (13) of said applanation cone being configured to be mounted with translational mobility in a direction parallel to an optical axis (8) of the focusing optical block on said fixing ring (17).

4. The applanation device of claim 3, further comprising an angular orientation ring (15) configured to be mounted with rotational mobility in a plane (XY) at right angles to the optical axis (8) of the focusing optical block on said protruding part, the upper part (13) of said applanation cone being configured to be mounted with translational mobility in a direction parallel to the optical axis of the focusing optical block on said angular orientation ring (15).

5. The applanation device as claimed in one of the preceding claims, wherein the applanation cone (11) comprises at least one camera-holding element (27, 28) fixed onto the outer wall of the applanation cone (11) and at least one side aperture (20) arranged with respect to said holding element so as to allow the passage of an imaging beam generated by said at least one camera to view a cutting zone of the eye situated under the applanation glass (12).

6. The applanation device as claimed in claim 5, wherein said holding element (27, 28) is configured such that the imaging beam generated by said camera is inclined by an angle of between 30° and 50°, preferably between 45° and 47°, with respect to the optical axis (8) of the focusing optical block.

7. The applanation device as claimed in one of the preceding claims, wherein the applanation glass (12) comprises at least one pattern or a graduated rule produced on the surface or in the volume.

8. The applanation device as claimed in one of the preceding claims, wherein the applanation glass has a planar-planar (32) or planar-concave (33) geometry.

9. The applanation device as claimed in one of claims 3 to 8, wherein the upper part of said applanation cone (11) comprises an axial displacement ring (13) provided with an inner threading, designed to be engaged by screwing with an outer threading produced on the outer wall of the fixing ring (17) or of the orientation ring (15), said axial displacement ring (13) ensuring, in a screwing step, an axial displacement of said applanation cone (11) in a direction parallel to the optical axis (8) of the focusing optical block.

10. The applanation device as claimed in one of claims 3 to 9, further comprising a locking ring (16) configured to lock the applanation cone (11) in position.

11. The applanation device as claimed in one of claims 1 to 10, wherein the applanation cone is produced in a metallic material by additive manufacturing.

12. The applanation device as claimed in one of claims 1 to 11, wherein the applanation cone comprises a lower part (14) fixed onto the upper part (13), said lower part having an upside-down truncated cone form.

13. An applanation assembly (50) for holding an eye of a patient in position and creating a reference plane for a laser system for a laser ophthalmological surgery operation, said assembly comprising:
- an applanation device (10) as claimed in one of claims 1 to 12;
- a suction ring (40) designed to come into contact with a surface of the eye of the patient;
- a portion of said lower part of the applanation cone being configured to be inserted into the suction ring to position the applanation glass inside the suction ring.

14. An ophthalmological surgery appliance (100) for making a cut in an ocular biological tissue, such as a cornea or a lens, comprising:
- a laser unit (104) designed to deliver a laser beam,
- a focusing optical block (105) for focusing the laser beam at a focal point in the ocular biological tissue;
- said focusing optical block comprising a protruding lower part (106) extending from a bottom surface of the laser unit,
- an applanation device (10) as claimed in one of claims 1 to 12 configured to be fixed onto the protruding lower part of the focusing optical block so as to position the applanation glass at a distance substantially close to the focusing distance with respect to the optical output of the focusing optical block.

15. The appliance as claimed in claim 14, further comprising at least one camera (107, 108) configured to view the cutting zone, said at least one camera being held in position on the outer wall of the applanation cone.

16. The appliance as claimed in one of claims 14 and 15, further comprising a centering camera (109) configured for the centering of the optical axis of symmetry of the focusing system with respect to the biological tissue, one end of the centering camera being inserted into the applanation cone via one of the two apertures provided in the applanation cone.
